(19) Europäisches Patentamt / European Patent Office / Office européen des brevets

(11) **EP 1 800 746 A1**

(12) **DEMANDE DE BREVET EUROPEEN**

(43) Date de publication:
**27.06.2007 Bulletin 2007/26**

(51) Int Cl.:
***B01J 29/80*** (2006.01)     ***B01J 29/44*** (2006.01)
***C07C 5/27*** (2006.01)

(21) Numéro de dépôt: **06291722.4**

(22) Date de dépôt: **03.11.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **22.12.2005 FR 0513157**

(71) Demandeur: **Institut Français du Pétrole**
**92852 Rueil Malmaison Cédex (FR)**

(72) Inventeurs:
• **Guillon, Emmanuelle**
  **69390 Vernaison (FR)**
• **Sanchez, Eric**
  **69230 Saint Genis Lavalq (FR)**
• **Lacombe, Sylvie**
  **69230 Saint Genis Laval (FR)**

(54) **Catalyseur comprenant une zeolithe de type structural nes et une zeolithe de type structural euo et son utilisation en isomerisation des composes C8 aromatiques**

(57) On décrit un catalyseur comportant au moins une zéolithe de type structural EUO, au moins une zéolithe de type structural NES, au moins un métal choisi parmi les métaux des groupes IIIA, VIB, VIIB et VIII et au moins une matrice minérale poreuse. Le catalyseur selon l'invention est utilisé dans un procédé d'isomérisation d'une charge comprenant des composés aromatiques à 8 atomes de carbone par molécule.

EP 1 800 746 A1

**Description**

Domaine technique

La présente invention se rapporte à un catalyseur formé d'au moins deux zéolithes, l'une de type structural EUO et l'autre de type structural NES, utilisable par exemple dans les réactions de transformation des hydrocarbures aromatiques. De manière plus précise, elle concerne un catalyseur d'isomérisation des composés C8 aromatiques. La présente invention concerne aussi l'utilisation dudit catalyseur dans un procédé d'isomérisation d'une charge comprenant des composés aromatiques à 8 atomes de carbone par molécule.

Art antérieur

Selon les procédés connus d'isomérisation des composés aromatiques à huit atomes de carbone (AC8), une charge généralement pauvre en paraxylène par rapport à l'équilibre thermodynamique du mélange (c'est-à-dire dont la teneur en paraxylène est nettement inférieure à celle du mélange à l'équilibre thermodynamique à la température considérée, ce mélange comprenant au moins un composé choisi dans le groupe formé par le métaxylène, l'orthoxylène, le paraxylène et l'éthylbenzène) et généralement riche en éthylbenzène par rapport à ce même mélange à l'équilibre thermodynamique est introduite dans un réacteur contenant au moins un catalyseur, dans des conditions de température et de pression adéquates pour obtenir une composition, à la sortie dudit réacteur, en composés aromatiques à huit atomes de carbone la plus proche possible de la composition dudit mélange à l'équilibre thermodynamique à la température du réacteur. Pour obtenir une telle composition, l'Homme du métier s'efforce généralement de maximiser la conversion en éthylbenzène présent dans la charge. A partir du mélange obtenu en sortie du réacteur d'isomérisation, on sépare le xylène et éventuellement le métaxylène ou l'orthoxylène qui sont les isomères recherchés car ils présentent un intérêt important notamment pour l'industrie des fibres synthétiques.

Les catalyseurs utilisés pour la mise en oeuvre d'un procédé d'isomérisation de composés aromatiques à huit atomes de carbone sont généralement des catalyseurs zéolithiques. Les catalyseurs de l'état de la technique, en particulier les catalyseurs à base de zéolithe mordénite, ne donnent que des performances catalytiques médiocres car ils conduisent à des réactions parasites non négligeables générant des pertes. Par exemple, on peut citer, parmi ces réactions secondaires, l'ouverture de cycles naphténiques suivie ou non de craquage (pertes vers les paraffines) ou encore les réactions de dismutation et de transalkylation des aromatiques à huit atomes de carbone (pertes vers des composés aromatiques non recherchés) ou bien encore l'hydrogénation des composés aromatiques (pertes vers les naphtènes). Des catalyseurs à base de zéolithe ZSM-5, seule ou en mélange avec d'autres zéolithes comme la mordénite par exemple, ont déjà été utilisés mais ne donnent pas, non plus, des performances catalytiques optimales. Plus récemment, il a été proposé un catalyseur à base d'une zéolithe de type structural EUO (EP-A1-0.923.987). Aussi, la présente invention se propose de fournir un nouveau catalyseur présentant une composition telle que lorsqu'il est utilisé pour l'isomérisation des composés aromatiques à 8 atomes de carbone par molécule, la conversion en éthylbenzène est améliorée et les réactions secondaires sont limitées, réduisant en conséquence les pertes.

Résumé

La présente invention a pour objet un catalyseur comportant au moins une zéolithe de type structural NES, au moins une zéolithe de type structural EUO, au moins un métal choisi parmi les métaux des groupes VIII, VIIB, VIB et IIIA et au moins une matrice minérale poreuse. De manière avantageuse, le catalyseur selon la présente invention comporte en outre éventuellement au moins un métal choisi parmi les métaux du groupe IVA. Chacune des zéolithes comprises dans le catalyseur selon l'invention contient du silicium et au moins un élément T choisi dans le groupe formé par l'aluminium, le fer, le gallium et le bore, de préférence l'aluminium.

La présente invention concerne également l'utilisation dudit catalyseur dans un procédé d'isomérisation d'une charge comprenant des composés aromatiques à 8 atomes de carbone par molécule.

Intérêt

Il a été découvert, de manière surprenante, qu'un catalyseur composite comprenant l'association d'au moins une zéolithe de type structural NES et d'au moins une zéolithe de type structural EUO ainsi qu'au moins un métal choisi parmi les métaux des groupes IIIA, VIB, VIIB et VIII conduit à des performances catalytiques améliorées dans les réactions d'isomérisation des composés aromatiques à 8 atomes de carbone par molécule. En particulier, le catalyseur selon l'invention conduit à une conversion en éthylbenzène supérieure à celle réalisée par les catalyseurs de l'état de la technique, notamment des catalyseurs à base d'une seule zéolithe de type structural EUO ou d'une seule zéolithe de type structural MOR. Par ailleurs, le catalyseur selon l'invention limite notablement les réactions secondaires, générant ainsi de moindres pertes, par rapport aux catalyseurs de l'état de la technique.

De plus, en ajustant la quantité relative des deux zéolithes, celle à type structural EUO et celle à type structural NES, dans le catalyseur selon l'invention, il est possible de traiter une très large gamme de mélanges de charges hydrocarbonées.

Description

La présente invention a pour objet un catalyseur comportant au moins une zéolithe de type structural EUO, au moins une zéolithe de type structural NES, au moins un métal choisi parmi les métaux des groupes IIIA, VIB, VIIB et VIII et au moins une matrice minérale poreuse.

La zéolithe de type structural EUO et la zéolithe de type structural NES présentes dans le catalyseur selon l'invention comprennent du silicium et au moins un élément T choisi dans le groupe formé par l'aluminium, le fer, le gallium et le bore, de préférence l'aluminium. Elles sont, de préférence, pratiquement totalement, sous forme acide.

La zéolithe de type structural EUO, présente dans le catalyseur selon l'invention, est déjà décrite dans l'état de la technique. Elle présente un réseau microporeux monodimensionnel, dont le diamètre des pores est de 4,1 x 5,7 Å (1 Å = 1 Angström = $10^{-10}$ m) (« Atlas of Zeolite Framework Types », W.M. Meier, D.H. Olson, Ch. Baerlocher, 5ème Edition, 2001). D'autre part, N.A. Briscoe et al ont enseigné dans un article de la revue Zeolites (1988, 8, 74) que ces canaux monodimensionnels possèdent des poches latérales de profondeur 8,1 Å et de diamètre 6,8 x 5,8 Å. La zéolithe de type structural EUO regroupe les zéolithes EU-1 (EP-B1-42 226), ZSM-50 (US 4.640.829) et TPZ-3 (EP-A1-51 318). La zéolithe de type structural EUO, présente dans le catalyseur selon l'invention, est de préférence une zéolithe EU-1. Ladite zéolithe de type structural EUO est caractérisée par un rapport atomique Si/T, de préférence un rapport atomique Si/Al, d'au moins 5, avantageusement compris entre 5 et 100. Ladite zéolithe de type structural EUO est au moins en partie, de préférence pratiquement totalement, sous forme acide, c'est-à-dire sous forme hydrogène H$^+$, la teneur en sodium étant de préférence telle que le rapport atomique Na/T est inférieur à 0,1, de manière plus préférée inférieur à 0,05. Un mode de synthèse d'une zéolithe EU-1 est décrit dans le brevet EP-B1-42 226. Un mode de synthèse d'une zéolithe ZSM-50 est décrit dans le brevet US 4.640.829. Un mode de synthèse d'une zéolithe TPZ-3 est décrit dans la demande de brevet EP-A1-51 318.

La zéolithe de type structural NES, comprise dans le catalyseur selon la présente invention, est répertoriée dans l'Atlas des Zéolithes (« Atlas of Zeolite Framework Types », W.M. Meier, D.H. Olson et Ch. Baerlocher, 5ème Edition, 2001). De préférence, il s'agit d'une zéolithe NU-87. Ladite zéolithe de type structural NES, de préférence une zéolithe NU-87, est caractérisée par un rapport atomique Si/T, de préférence un rapport atomique Si/Al, compris entre 2 et 250, de préférence compris entre 5 et 150 et de manière très préférée compris entre 10 et 80. La zéolithe de type structural NES, préférentiellement une zéolithe NU-87, est de préférence telle que de l'élément T, avantageusement de l'aluminium, a été extrait de la charpente. La teneur en sodium est inférieure à 0,2 % poids, de préférence inférieure à 0,1 % poids et de manière très préférée inférieure à 0,05 % poids par rapport au poids total de zéolithe sèche. Ladite zéolithe de type structural NES est synthétisée selon les méthodes décrites dans les références citées dans l'Atlas des Zéolithes ou par toute autre méthode décrite dans la littérature ouverte à l'Homme du métier. En particulier, une zéolithe NU-87 peut être préparée en mélangeant au moins une source de silicium, au moins une source d'un élément T choisi dans le groupe formé par l'aluminium, le fer, le gallium et le bore, au moins un cation alcalin et au moins un structurant organique choisi parmi les sels de polyméthylène diammonium, par exemple le bromure de décaméthonium. Une méthode de préparation d'une zéolithe de type structural NES, de préférence d'une zéolithe NU-87, est donnée dans les brevets EP 0.377.291, EP 0.378.916 B1 et US 5.041.402, le contenu de chacun de ces brevets étant intégré par référence dans la présente description.

Les rapports atomiques Si/T, de préférence les rapports atomiques Si/Al, des zéolithes de type structural NES et EUO décrites ci-dessus, sont ceux obtenus à l'issue de la synthèse desdites zéolithes ou bien obtenus après des traitements post-synthèse d'extraction d'une partie des atomes T, dits traitements de désalumination lorsque T est l'aluminium, bien connus de l'homme du métier, tels que et à titre non exhaustif les traitements hydrothermiques suivis ou non d'attaques acides ou bien encore les attaques acides directes par des solutions d'acides minéraux ou organiques visant à extraire une partie des atomes T, de préférence une partie des atomes d'aluminium, de la charpente zéolithique. De préférence, la zéolithe de type structural NES présente dans le catalyseur selon l'invention a été obtenue par désalumination post-synthèse.

Le rapport atomique Si/T, de préférence le rapport atomique Si/Al, de la zéolithe de type structural EUO et de la zéolithe de type structural NES entrant dans la composition du catalyseur selon l'invention ainsi que la composition chimique dudit catalyseur sont déterminés par fluorescence X et absorption atomique.

Les zéolithes de type structural NES et EUO entrant dans la composition du catalyseur selon l'invention peuvent être calcinées et échangées par au moins un traitement par une solution d'au moins un sel d'ammonium de manière à obtenir la forme ammonium des zéolithes qui une fois calcinée conduisent à la forme hydrogène desdites zéolithes.

Les zéolithes de type structural NES et EUO entrant dans la composition du catalyseur selon l'invention sont au moins en partie, de préférence pratiquement totalement, sous forme acide, c'est-à-dire sous forme hydrogène (H$^+$). Le rapport atomique Na/T est généralement inférieur à 10%, de préférence inférieur à 5% et de manière encore plus préférée inférieur à 1 %.

Ledit catalyseur selon l'invention comprend en outre au moins un métal choisi parmi les métaux des groupes IIIA, VIB, VIIB et VIII, de préférence choisi parmi les métaux des groupes VIIB et VIII, de manière très préférée ledit métal est choisi parmi les métaux du groupe VIII. Ledit métal est présent à une teneur comprise entre 0,01 et 5 % poids par rapport au poids total du catalyseur. Parmi les métaux du groupe VIIB, le rhénium est préféré. Parmi les métaux du groupe VIII, le platine est préféré. Parmi les métaux du groupe IIIA, le gallium est préféré. Parmi les métaux du groupe VIB, le molybdène est préféré. Un catalyseur préféré selon l'invention comprend au moins une zéolithe NU-87, au moins une zéolithe EU-1, au moins du platine et au moins une matrice minérale poreuse, par exemple de l'alumine. Un autre catalyseur préféré selon l'invention comprend au moins une zéolithe NU-87, au moins une zéolithe EU-1, au moins du platine, au moins du rhénium et au moins une matrice minérale poreuse, par exemple de l'alumine. Le catalyseur selon l'invention comporte éventuellement en outre au moins un métal additionnel choisi parmi les métaux du groupe IVA, de préférence de l'étain. Ledit métal additionnel est présent, de préférence, à une teneur comprise entre 0,01 et 5 % poids et de manière très préférée entre 0,5 et 3 % poids par rapport au poids total du catalyseur.

La matrice minérale poreuse, présente à une teneur pondérale comprise entre 5 et 98 %, de préférence entre 20 et 95 %, de manière plus préférée entre 30 et 92% par rapport au poids total de catalyseur, est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium, les silice-alumines amorphes et le charbon, de préférence parmi les éléments du groupe formé par les alumines et les argiles, de manière encore plus préférée parmi les alumines, notamment l'alumine gamma.

Selon une première variante de préparation du catalyseur de l'invention, préalablement à leur mise en forme, au moins une des zéolithes précédemment décrites, c'est-à-dire au moins une zéolithe de type structural NES ou au moins une zéolithe de type structural EUO, comprise dans ledit catalyseur est soumise au dépôt d'au moins un métal choisi parmi les métaux des groupes IIIA, VIB, VIIB et VIII, et éventuellement au dépôt d'au moins un métal du groupe IVA. Il est aussi possible que la zéolithe de type structural NES soit soumise au dépôt d'un métal choisi parmi les métaux des groupes IIIA, VIB, VIIB et VIII, de préférence parmi les métaux du groupe VIIB, de manière très préférée le rhénium, et que la zéolithe de type structural EUO soit soumise au dépôt d'un autre métal choisi parmi les métaux des groupes IIIA, VIB, VIIB et VIII, de préférence parmi les métaux du groupe VIII, de manière très préférée le platine. Les zéolithes, dont l'une au moins est chargée en métal(ux), sont mélangées. Le mélange de ces zéolithes, dont l'une au moins est chargée en métal(ux) et qui sont à l'état de poudre est réalisé par toutes les techniques de mélange de poudres connues de l'homme du métier.

Une fois le mélange des poudres de zéolithes, dont l'une au moins est chargée en métal(ux), réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ces formes connues de l'Homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées. Après l'étape de mise en forme, le produit obtenu est soumis à une étape de séchage réalisée à une température comprise entre 80 et 150°C puis à une étape de calcination réalisée à une température comprise entre 300 et 600°C, de préférence entre 400 et 550°C.

Selon une deuxième variante de préparation du catalyseur selon l'invention, au moins un métal choisi parmi les métaux des groupes IIIA, VIB, VIIB et VIII et éventuellement au moins un métal choisi parmi les métaux du groupe IVA est(sont) déposé(s) sur le support catalytique après la mise en forme des zéolithes de type structural NES et EUO exemptes de métaux, par tout procédé connu de l'homme du métier et permettant le dépôt du métal ou des métaux sur le support catalytique. On désigne par le terme "support", le mélange des zéolithes (exemptes de métaux) avec au moins une matrice minérale poreuse après mise en forme, séchage à une température comprise entre 80 et 150°C et calcination à une température comprise entre 300 et 600°C, de préférence entre 400 et 550°C. Initialement, les zéolithes de type structural NES et EUO se trouvent à l'état de poudre, le mélange desdites zéolithes est réalisé par toutes les techniques de mélange de poudres connues de l'Homme du métier. Une fois le mélange des poudres de zéolithes, réalisé, le mélange est mis en forme par toute technique connue de l'Homme du métier. Il peut en particulier être mélangé à une matrice minérale poreuse, généralement amorphe, par exemple à une poudre humide de gel d'alumine. Le mélange est ensuite mis en forme, par exemple par extrusion au travers d'une filière. La mise en forme peut être réalisée avec d'autres matrices que l'alumine, en particulier par une matrice choisie parmi les éléments du groupe décrit plus haut dans la présente description. Le support du catalyseur de la présente invention renferme généralement les teneurs suivantes en matrice et zéolithes :

- 2 à 95% poids, de préférence de 5 à 80% poids, de manière plus préférée de 8 à 70% poids de zéolithes de type structural NES et EUO,
- 5 à 98% poids, de préférence 20 à 95% poids, de manière plus préférée de 30 à 92% poids d'au moins une matrice minérale poreuse amorphe ou mal cristallisée de type oxyde.

Pour le dépôt de métal(ux) sur au moins une des zéolithes et/ou sur le support catalytique selon la première ou la deuxième variante de préparation du catalyseur selon l'invention, on peut utiliser la technique d'échange cationique avec compétition où le compétiteur est de préférence le nitrate d'ammonium, le rapport de compétition entre le compétiteur et le précurseur métallique étant au moins égal à environ 5 et avantageusement compris entre 5 et 200. On peut utiliser aussi la technique d'imprégnation à sec ou de coprécipitation.

Les sources des métaux du groupe VIII qui peuvent être utilisées sont bien connues de l'Homme du métier. Par exemple, on utilisera les nitrates, les sulfates, les phosphates, les halogénures par exemples chlorures, bromures et fluorures, les carboxylates par exemple acétates et carbonates. Dans le cas du platine, on utilise préférentiellement l'acide hexachloroplatinique ou le chlorure de platine tétramine. Dans le cas du nickel, on peut utiliser préférentiellement du nitrate de nickel $Ni(NO_3)_2$. Les sources des métaux du groupe VIIB qui peuvent être utilisées sont également bien connues de l'Homme du métier. Dans le cas du rhénium, on utilise habituellement un complexe de perrhénate d'ammonium $(NH_4)ReO_4$ ou l'acide perrhénique. Les sources des métaux du groupe IIIA qui peuvent être utilisées sont également bien connues de l'Homme du métier. Dans le cas du gallium, le nitrate de gallium $Ga(NO_3)_3$ est préféré. Les sources des métaux du groupe VIB qui peuvent être utilisées sont également bien connues de l'Homme du métier. Dans le cas du molybdène, on peut utiliser les acides molybdiques et leurs sels en particulier les sels d'ammonium tels que le molybdate d'ammonium, l'heptamolybdate d'ammonium ainsi que l'acide phosphomolybdique. De préférence, on utilise l'hepta-molybdate d'ammonium $(NH_4)_6Mo_7O_{24}$. Le dépôt du métal ou des métaux des groupes IIIA, VIB, VIIB et VIII et éventuellement du groupe IVA est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures, de préférence on opérera entre 350° et 550°C pendant 2 à 5 heures.

Selon la première et la deuxième variante de préparation du catalyseur selon l'invention, on peut également déposer le métal ou les métaux non plus directement sur les zéolithes, mais sur la matrice minérale poreuse (par exemple le liant aluminique) du support, avant ou après l'étape de mise en forme, en mettant en oeuvre un échange anionique. On peut citer par exemple dans le cas du dépôt de platine l'utilisation du complexe hexachloroplatinique $H_2PtCl_6$ et dans le cas du dépôt de rhénium l'utilisation de l'acide perrhénique $HReO_4$. En général après le dépôt de métal, le catalyseur est comme précédemment soumis à une calcination puis réduit sous hydrogène comme indiqué ci-dessus.

Dans le cas où le catalyseur contient plusieurs métaux, ces derniers peuvent être introduits soit tous de la même façon soit par des techniques différentes, avant ou après mise en forme selon la variante de préparation du catalyseur employée et dans n'importe quel ordre. Dans le cas où la technique utilisée est celle de l'échange ionique, plusieurs échanges successifs peuvent être nécessaires pour introduire les quantités requises de métaux.

Quelle que soit la variante de préparation du catalyseur selon l'invention, on peut procéder après la calcination dudit catalyseur à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu ex situ ou in situ, par rapport au lieu d'utilisation dudit catalyseur dans une réaction donnée.

La répartition entre les deux zéolithes présentes dans le catalyseur selon l'invention est telle que la teneur en zéolithe de type structural EUO peut varier de 1% à 99%, de préférence de 5 à 95% et de manière encore plus préférée de 10 à 90% en pourcentages massiques de la zéolithe de type structural EUO par rapport à l'ensemble des zéolithes introduites dans le catalyseur. De même, la teneur en zéolithe de type structural NES varie de 1% à 99%, de préférence de 5 à 95% et de manière encore plus préférée de 10 à 90%, en pourcentages massiques de la zéolithe de type structural NES par rapport à l'ensemble des zéolithes introduites dans le catalyseur.

Le catalyseur selon la présente invention est mis en forme sous la forme de grains de différentes formes et dimensions. Il est utilisé en général sous la forme d'extrudés cylindriques ou polylobés tels que bilobés, trilobés, polylobés de forme droite ou torsadée, mais peut éventuellement être fabriqué et employé sous la forme de poudre, pastilles, de tablettes, d'anneaux, de billes, de roues.

Le catalyseur de la présente invention peut éventuellement contenir du soufre. Dans ce cas, le soufre est introduit sur le catalyseur mis en forme, calciné, contenant le ou les élément(s) cité(s) précédemment, soit *in situ* avant la réaction catalytique, soit *ex situ.* La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Un autre objet de l'invention est l'utilisation du catalyseur selon l'invention dans des procédés pour la conversion d'hydrocarbures. Plus précisément, la présente invention concerne un procédé d'isomérisation d'une charge comprenant des composés aromatiques à 8 atomes de carbone par molécule réalisé en présence du catalyseur selon l'invention.

Ladite charge comprend un mélange de xylènes et de l'éthylbenzène. De manière préférée, ladite charge est dépourvue de naphtalène et de manière très préférée elle est dépourvue de naphtalène et de composés aromatiques ayant au moins 11 atomes de carbone par molécule. Ledit procédé est mis en oeuvre généralement selon les conditions opératoires suivantes :

- une température comprise entre 300°C et 500°C, de préférence entre 320°C et 450°C et de manière encore plus préférée entre 340°C et 430°C,
- une pression partielle d'hydrogène comprise entre 0,3 et 1,5 MPa, de préférence entre 0,4 et 1,2 MPa et de manière encore préférée entre 0,7 et 1,2 MPa,
- une pression totale comprise entre 0,45 et 1,9 MPa, de préférence entre 0,6 et 1,5 MPa,
- une vitesse spatiale d'alimentation, exprimée en kilogramme de charge introduite par kilogramme de catalyseur et par heure, comprise entre 0,25 et 30 $h^{-1}$, de préférence entre 1 et 10 $h^{-1}$, et de manière encore plus préférée entre 2 et 6 $h^{-1}$.

Les exemples qui suivent illustrent l'invention sans pour autant en limiter la portée.

**Exemple 1 : Préparation d'un catalyseur à base d'une zéolithe EU-1 (comparatif).**

La matière première utilisée est une zéolithe EU-1, brute de synthèse, comprenant le structurant organique, du silicium et de l'aluminium, possédant un rapport atomique Si/Al global égal à 13,6, une teneur pondérale en sodium par rapport au poids en zéolithe EU-1 sèche d'environ 1,5%, correspondant à un rapport atomique Na/Al de 0,6. Cette zéolithe EU-1 subit tout d'abord une calcination dite sèche à 550°C sous flux d'air durant 6 heures. Puis le solide obtenu est soumis à trois échanges ioniques dans une solution de $NH_4NO_3$ 10N, à environ 100°C pendant 4 heures pour chaque échange. A l'issue de ces traitements, la zéolithe EU-1 sous forme $NH_4$ a un rapport Si/Al atomique global égal à 18,3, une teneur pondérale en sodium par rapport au poids de zéolithe EU-1 sèche de 50 ppm, correspondant à un rapport atomique Na/Al de 0,003. La zéolithe EU-1 est ensuite mise en forme par extrusion avec un gel d'alumine de manière à obtenir, après séchage et calcination sous air sec, le support constitué d'extrudés de 1,4 mm de diamètre, qui contient en poids 15 % de zéolithe EU-1 sous forme H et 85 % d'alumine.

Le support ainsi obtenu est soumis à un échange anionique avec de l'acide hexachloroplatinique en présence d'un agent compétiteur (acide chlorhydrique), de manière à introduire 0,3% poids de platine par rapport au poids du catalyseur. Le solide humide est ensuite séché à 120°C pendant 12 heures et calciné sous un débit d'air sec à la température de 500°C pendant une heure. Le catalyseur A ainsi obtenu contient en poids 15,0% de zéolithe EU-1 sous forme H, 84,7% d'alumine et 0,3% de platine.

**Exemple 2 : Préparation d'un catalyseur à base d'une zéolithe NU-87 (comparatif).**

La zéolithe NU-87 a été synthétisée d'après les enseignements des brevets européen EP-B-0.377.291 ou EP-B-0.378.916. Elle possède un rapport atomique Si/Al global égal à 17,2, une teneur pondérale en sodium égale à 1256 ppm. Cette zéolithe NU-87 subit, tout d'abord, une calcination dite sèche à 550°C sous flux d'air et d'azote durant 6 heures. Puis, le solide obtenu est soumis à un échange ionique dans une solution de $NH_4NO_3$ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires. A l'issue de ces traitements, la zéolithe obtenue se trouve sous sa forme H et possède un rapport Si/Al atomique global égal à 33,3, et une teneur en Na de 10 ppm.

La zéolithe NU-87 est ensuite mise en forme par extrusion avec un gel d'alumine de manière à obtenir, après séchage et calcination sous air sec, le support constitué d'extrudés de 1,4 mm de diamètre, qui contient en poids 15 % de zéolithe NU-87 sous forme H et 85 % d'alumine.

Le support ainsi obtenu est soumis à un échange anionique avec de l'acide hexachloroplatinique en présence d'un agent compétiteur (acide chlorhydrique), de manière à introduire 0,3% poids de platine par rapport au poids du catalyseur. Le solide humide est ensuite séché à 120°C pendant 12 heures et calciné sous un débit d'air sec à la température de 500°C pendant une heure. Le catalyseur B ainsi obtenu contient en poids 15,0% de zéolithe NU-87 sous forme H, 84,7%

d'alumine et 0,3% de platine.

**Exemple 3 : préparation d'un catalyseur à base d'une zéolithe NU-87 et d'une zéolithe EU-1 (invention).**

La zéolithe EU-1 est synthétisée de la même manière que la procédure donnée à l'exemple 1. A l'issue de l'étape de synthèse, la zéolithe EU-1 se présente sous forme $NH_4$, elle présente un rapport Si/Al atomique égal à 18,3, une teneur pondérale en sodium par rapport au poids de zéolithe EU-1 sèche de 50 ppm, correspondant à un rapport atomique Na/Al de 0,003.
La zéolithe NU-87 est synthétisée de la même manière que la procédure donnée à l'exemple 2. A l'issue de l'étape de synthèse, la zéolithe NU-87 se trouve sous sa forme H et possède un rapport Si/Al atomique égal à 33,3, et une teneur en Na de 10 ppm.
La zéolithe EU-1 et la zéolithe NU-87, qui se trouvent à l'état de poudre, sont mélangées mécaniquement puis mises en forme par extrusion avec un gel d'alumine de manière à obtenir, après séchage à 120°C pendant une nuit et calcination à 500°C sous air sec, un support qui contient en poids 15 % de zéolithes EU-1 et NU-87 et 85 % d'alumine.
Le support ainsi obtenu est soumis à un échange anionique avec de l'acide hexachloroplatinique en présence d'un agent compétiteur (acide chlorhydrique), de manière à déposer 0,3 % poids de platine par rapport au poids du catalyseur. Le solide humide est ensuite séché à 120°C pendant 12 heures et calciné sous un débit d'air sec à 500°C pendant une heure. On obtient ainsi le catalyseur C contenant en poids, 8,0 % de zéolithe EU-1 forme hydrogène, 7,0% de zéolithe NU-87 forme H, 84,7 % d'alumine et 0,3 % de platine.

**Exemple 4 : préparation d'un catalyseur à base d'une zéolithe NU-87 et d'une zéolithe EU-1 (invention).**

La zéolithe EU-1 est synthétisée de la même manière que la procédure donnée à l'exemple

1. A l'issue de l'étape de synthèse, la zéolithe EU-1 se présente sous forme $NH_4$, elle présente un rapport Si/Al atomique égal à 18,3, une teneur pondérale en sodium par rapport au poids de zéolithe EU-1 sèche de 50 ppm, correspondant à un rapport atomique Na/Al de 0,003. La zéolithe EU-1 est soumise à une imprégnation à sec par une solution d'acide hexachloroplatinique de manière à déposer 0,15 % poids de platine par rapport au poids du catalyseur final. Le solide humide est ensuite séché à 120°C pendant 12 heures et calciné sous un débit d'air sec à 500°C pendant 1 heure.

La zéolithe NU-87 est synthétisée de la même manière que la procédure donnée à l'exemple 2. A l'issue de l'étape de synthèse, la zéolithe NU-87 se trouve sous sa forme H et possède un rapport Si/Al atomique-égal à 33,3, et une teneur en Na de 10 ppm. La zéolithe NU-87 est soumise à une imprégnation à sec par une solution de perrhénate d'ammonium de manière à déposer 0,15 % poids de rhénium par rapport au poids du catalyseur final. Le solide humide est ensuite séché à 120°C pendant 12 heures et calciné sous un débit d'air sec à 500°C pendant 1 heure.
La zéolithe EU-1 imprégnée de platine et la zéolithe NU-87 imprégnée de rhénium sont mélangées puis mises en forme par extrusion avec un gel d'alumine de manière à obtenir, après séchage à 120°C pendant une nuit et calcination à 500°C sous air sec, le catalyseur D contenant en poids, 8,0 % de zéolithe EU-1 forme hydrogène, 7,0% de zéolithe NU-87 forme H, 84,7 % d'alumine, 0,15 % de platine et 0,15 % de rhénium.

**Exemple 5 : préparation d'un catalyseur à base d'une zéolithe EU-1 et d'une zéolithe ZSM-22 (comparatif).**

La zéolithe EU-1 est synthétisée de la même manière que la procédure donnée à l'exemple 1. A l'issue de l'étape de synthèse, la zéolithe EU-1 se présente sous forme $NH_4$, elle présente un rapport Si/Al atomique égal à 18,3, une teneur pondérale en sodium par rapport au poids de zéolithe EU-1 sèche de 50 ppm, correspondant à un rapport atomique Na/Al de 0,003.
La zéolithe ZSM-22 est synthétisée de la même manière que la procédure donnée dans l'article de Marler B, publié dans *Zeolites,* 7(1987)2327. A l'issue de l'étape de synthèse, la zéolithe ZSM-22 se trouve sous sa forme H et possède un rapport Si/Al atomique égal à 33, et une teneur en Na de 86 ppm.
La zéolithe EU-1 et la zéolithe ZSM-22, qui se trouvent à l'état de poudre, sont mélangées mécaniquement puis mises en forme par extrusion avec un gel d'alumine de manière à obtenir, après séchage à 120°C pendant une nuit et calcination à 500°C sous air sec, un support qui contient en poids 15 % de zéolithes EU-1 et ZSM-22 et 85 % d'alumine.
Le support ainsi obtenu est soumis à un échange anionique avec de l'acide hexachloroplatinique en présence d'un agent compétiteur (acide chlorhydrique), de manière à déposer 0,3 % poids de platine par rapport au poids du catalyseur. Le solide humide est ensuite séché à 120°C pendant 12 heures et calciné sous un débit d'air sec à 500°C pendant une heure. On obtient ainsi le catalyseur E contenant en poids, 8,0 % de zéolithe EU-1 forme hydrogène, 7,0% de zéolithe ZSM-22 forme H, 84,7 % d'alumine et 0,3 % de platine.

**Exemple 6 : Evaluation des propriétés catalytiques des catalyseurs A, B, C, D et E en isomérisation d'une coupe C8 aromatique.**

Les performances des catalyseurs A, B, C, D et E ont été évaluées dans l'isomérisation d'une coupe aromatique comprenant des composés aromatiques à 8 atomes de carbone par molécule, principalement du métaxylène, de l'orthoxylène et de l'éthylbenzène. Les conditions opératoires sont les suivantes: - température : 390°C,

- pression totale : 15 bar, (1 bar = 0,1 MPa)
- pression partielle d'hydrogène: 12 bar.

Les catalyseurs sont préalablement traités avec une charge contenant du disulfure de diméthyle (DMDS) en présence d'hydrogène, avec une concentration telle que le rapport atomique soufre/métal soit de 1,5. Le catalyseur est ensuite maintenu pendant 3 heures à 400°C sous débit d'hydrogène puis la charge est injectée.

Les catalyseurs ont été comparés en terme d'activité par la conversion de l'éthylbenzène et en terme de sélectivité par les pertes nettes à iso-approche à l'équilibre du paraxylène.

La réaction d'isomérisation conduit à des réactions parasites générant trois types de pertes: les pertes vers les paraffines résultant essentiellement des réactions d'ouverture de cycles naphténiques suivies de craquage, les pertes vers les aromatiques formés par les réactions de dismutation et de transalkylation des aromatiques à 8 atomes de carbone (AC8), et enfin les pertes vers les naphtènes dont les naphtènes à 8 atomes de carbone (N8) dus à l'hydrogénation des aromatiques. Les N8 pouvant être recyclés, on comparera les pertes par craquage et dismutation/transalkylation incluant les naphtènes autres que N8 (dont la somme constitue les pertes nettes).

Les pertes par craquage (P1) sont des pertes en AC8 sous forme de paraffines (PAR) de C1 à C8 :

$$P1(\%poids) = 100 \ X \ [(\%PAR_{effluent} x poids \ d'effluent) - (\%PAR_{charge} x poids \ de \ charge)] \ / \ (\%AC8_{charge} x poids \ de \ charge)$$

Les pertes par dismutation/transalkylation (P2) sont des pertes en AC8 sous forme de naphtènes autres que N8, de toluène, de benzène et de C9+ aromatiques (OAN) :

$$P2(\%poids) = 100 \ X \ [(\%OAN_{effluent} x poids \ d'effluent) - (\%OAN_{charge} x poids \ de \ charge)] \ / \ (\%AC8_{charge} x poids \ de \ charge)$$

La somme des pertes P1 et P2 représente les pertes nettes.
Les données présentées dans le tableau 1 ont été obtenues à iso-conditions expérimentales et à iso-approche à l'équilibre (98%).

Tableau 1 : évaluation catalytique des catalyseurs A à E

| Catalyseur | A | B | C | D | E |
|---|---|---|---|---|---|
| conversion EB (%) | 61,3 | 62,8 | 63,3 | 64,6 | 59,6 |
| pertes nettes (% poids) | 6,2 | 6,3 | 5,0 | 4,8 | 7,2 |

Les catalyseurs C et D selon l'invention qui comprennent chacun une zéolithe de type structural EUO et une zéolithe de type structural NES, conduisent à une meilleure conversion de l'éthylbenzène et à une diminution des pertes nettes traduisant des réactions secondaires limitées par rapport aux performances obtenues au moyen du catalyseur A respectivement du catalyseur B uniquement à base d'une zéolithe de type structural EUO respectivement à base d'une zéolithe de type structural NES ou au moyen du catalyseur E qui ne comprend pas de zéolithe de type structural NES.

**Revendications**

1. Catalyseur comportant au moins une zéolithe de type structural EUO, au moins une zéolithe de type structural NES, au moins un métal choisi parmi les métaux des groupes IIIA, VIB, VIIB et VIII et au moins une matrice minérale poreuse.

2. Catalyseur selon la revendication 1 dans lequel la zéolithe de type structural EUO est une zéolithe EU-1.

3. Catalyseur selon la revendication 1 ou la revendication 2 dans lequel la zéolithe de type structural NES est une zéolithe NU-87.

4. Catalyseur selon l'une des revendications 1 à 3 dans lequel ledit métal choisi parmi les métaux des groupes IIIA, VIB, VIIB et VIII est choisi parmi les métaux des groupes VIIB et VIII.

5. Catalyseur selon l'une des revendications 1 à 4 comportant au moins une zéolithe NU-87, au moins une zéolithe EU-1, au moins du platine et au moins une matrice minérale poreuse.

6. Catalyseur selon l'une des revendications 1 à 4 comportant au moins une zéolithe NU-87, au moins une zéolithe EU-1, au moins du platine, au moins du rhénium et au moins une matrice minérale poreuse.

7. Catalyseur selon l'une des revendications 1 à 6 comportant au moins un métal additionnel choisi parmi les métaux du groupe IVA.

8. Catalyseur selon la revendication 7 tel que ledit métal additionnel est de l'étain.

9. Catalyseur selon l'une des revendications 1 à 8 comportant du soufre.

10. Procédé d'isomérisation d'une charge comprenant des composés aromatiques à 8 atomes de carbone par molécule réalisé en présence d'un catalyseur selon l'une des revendications 1 à 9.

11. Procédé d'isomérisation selon la revendication 10 tel que ladite charge comprend un mélange de xylènes et de l'éthylbenzène.

12. Procédé d'isomérisation selon la revendication 10 ou la revendication 11 tel qu'il est réalisé à une température comprise entre 300 et 500°C, avec une pression partielle d'hydrogène comprise entre 0,3 et 1,5 MPa, avec une pression totale comprise entre 0,45 et 1,9 MPa et une vitesse spatiale d'alimentation, exprimée en kilogramme de charge introduite par kilogramme de catalyseur et par heure, comprise entre 0,25 et 30 $h^{-1}$.

Office européen
des brevets

**RAPPORT DE RECHERCHE EUROPEENNE**

Numéro de la demande

EP 06 29 1722

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | EP 0 462 745 A (IMPERIAL CHEMICAL INDUSTRIES PLC) 27 décembre 1991 (1991-12-27) | 1-6, 10-12 | INV. B01J29/80 B01J29/44 C07C5/27 |
| Y | * revendications 1,2,4,10,11,13,16 * * abrégé * * page 2, ligne 15,16 * * page 7, ligne 49-55 * * page 28, ligne 43 - page 29, ligne 17 * * page 29, ligne 27-37 * * page 29, ligne 54 - page 30, ligne 27 * * exemples 25,28 * | 7-9 | |
| X | ----- US 2005/215838 A1 (NEGIZ ANTOINE ET AL) 29 septembre 2005 (2005-09-29) * revendication 21 * * page 3, alinéas 26,28,30 * * page 4, alinéa 30 * | 1-8 | |
| Y | ----- US 6 057 486 A (MERLEN ET AL) 2 mai 2000 (2000-05-02) * colonne 3, ligne 5-47 * * colonne 6, ligne 64 - colonne 7, ligne 16 * | 7-9 | |
| A | ----- US 4 537 754 A (CASCI ET AL) 27 août 1985 (1985-08-27) * abrégé * * colonne 6, ligne 12 - colonne 7, ligne 2 * | 1-12 | DOMAINES TECHNIQUES RECHERCHES (IPC) B01J C07C |
| A | ----- EP 0 378 916 A (IMPERIAL CHEMICAL INDUSTRIES PLC) 25 juillet 1990 (1990-07-25) * abrégé * * revendications 1-3,9,10 * ----- | 1-12 | |

Le présent rapport a été établi pour toutes les revendications

| Lieu de la recherche | Date d'achèvement de la recherche | Examinateur |
|---|---|---|
| Munich | 19 avril 2007 | Jourdan, Angelika |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**                    EP 06 29 1722

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-04-2007

| Document brevet cité<br>au rapport de recherche | | Date de<br>publication | Membre(s) de la<br>famille de brevet(s) | | Date de<br>publication |
|---|---|---|---|---|---|
| EP 0462745 | A | 27-12-1991 | AT | 133922 T | 15-02-1996 |
| | | | AU | 639110 B2 | 15-07-1993 |
| | | | AU | 7836991 A | 02-01-1992 |
| | | | CA | 2044960 A1 | 22-12-1991 |
| | | | CN | 1058195 A | 29-01-1992 |
| | | | CN | 1147422 A | 16-04-1997 |
| | | | DE | 69116937 D1 | 21-03-1996 |
| | | | DE | 69116937 T2 | 09-01-1997 |
| | | | DK | 462745 T3 | 17-06-1996 |
| | | | ES | 2085965 T3 | 16-06-1996 |
| | | | GR | 3019845 T3 | 31-08-1996 |
| | | | IN | 180874 A1 | 28-03-1998 |
| | | | JP | 3391805 B2 | 31-03-2003 |
| | | | JP | 7048122 A | 21-02-1995 |
| | | | US | 5385718 A | 31-01-1995 |
| | | | ZA | 9104542 A | 25-03-1992 |
| US 2005215838 | A1 | 29-09-2005 | CN | 1934058 A | 21-03-2007 |
| | | | EP | 1730093 A1 | 13-12-2006 |
| | | | WO | 2005097714 A1 | 20-10-2005 |
| US 6057486 | A | 02-05-2000 | AUCUN | | |
| US 4537754 | A | 27-08-1985 | DE | 3165380 D1 | 13-09-1984 |
| | | | EP | 0042226 A1 | 23-12-1981 |
| | | | US | 4593138 A | 03-06-1986 |
| EP 0378916 | A | 25-07-1990 | AU | 622964 B2 | 30-04-1992 |
| | | | AU | 4714689 A | 28-06-1990 |
| | | | CA | 2006542 A1 | 22-06-1990 |
| | | | CN | 1044052 A | 25-07-1990 |
| | | | DE | 68903560 D1 | 24-12-1992 |
| | | | DE | 68903560 T2 | 17-06-1993 |
| | | | DK | 654789 A | 23-06-1990 |
| | | | ES | 2044151 T3 | 01-01-1994 |
| | | | GR | 3006777 T3 | 30-06-1993 |
| | | | IN | 178832 A1 | 05-07-1997 |
| | | | JP | 2222727 A | 05-09-1990 |
| | | | JP | 2924911 B2 | 26-07-1999 |
| | | | NO | 895175 A | 25-06-1990 |
| | | | RU | 2067024 C1 | 27-09-1996 |
| | | | US | 5041402 A | 20-08-1991 |
| | | | ZA | 8909697 A | 27-03-1991 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- EP 0923987 A1
- EP 42226 B1
- US 4640829 A
- EP 51318 A1
- EP 0377291 A
- EP 0378916 B1
- US 5041402 A
- EP 0377291 B
- EP 0378916 B

**Littérature non-brevet citée dans la description**

- **W.M. MEIER ; D.H. OLSON ; CH. BAERLOCHER.** Atlas of Zeolite Framework Types. 2001
- **N.A. BRISCOE et al.** *Zeolites,* 1988, vol. 8, 74